## Europäisches Patentamt

## European Patent Office

## Office européen des brevets

(11) Publication number: **0 095 806**
**B1**

## EUROPEAN PATENT SPECIFICATION

(45) Date of publication of patent specification: **07.01.87**

(51) Int. Cl.⁴: **C 07 K 7/06,** C 07 K 1/06, C 07 B 45/02

(21) Application number: **83200726.4**

(22) Date of filing: **24.05.83**

(54) A process for preparing caerulein.

(30) Priority: **27.05.82 JP 90927/82**

(43) Date of publication of application: **07.12.83 Bulletin 83/49**

(45) Publication of the grant of the patent: **07.01.87 Bulletin 87/02**

(84) Designated Contracting States: **CH DE FR IT LI NL**

(56) References cited:
EP-A-0 103 538
FR-A-2 372 146
GB-A-2 016 478
US-A-3 057 855
US-A-3 472 832
US-A-3 892 726

CHEMICAL ABSTRACTS, vol. 72, no. 17, 1970, page 63, no. 86797m, Columbus, Ohio, USA L.R. JOHNSON et al.: "Effect of sulfation on the gastrointestinal actions of caerulein" Experentia 23, (1967), pp. 700-702

(73) Proprietor: **SHIONOGI & CO., LTD.**
**12, Dosho-machi 3-chome Higashi-ku Osaka 541 (JP)**

(72) Inventor: **Kikkawa, Ikuo**
**18-21, Tsurunoso**
**Takarazuka-shi Hyogo (JP)**
Inventor: **Uenaka, Masaaki**
**125, Nomaohara**
**Nose-cho Toyono-gun Osaka (JP)**

(74) Representative: **Bruin, Cornelis Willem et al**
**OCTROOIBUREAU ARNOLD & SIEDSMA P.O. Box 18558**
**NL-2502 EN The Hague (NL)**

Courier Press, Leamington Spa, England.

# 0 095 806

**Description**

This invention relates to a process of producing caerulein or its salts and to a sulfating agent useful in that process.

Caerulein is a decapeptide obtained from the skin of Hyla caerulea (an Australian amphibian). It can be used in medicine as a diagnostic agent for examining the function of pancreas and gallbladder. Its chemical structure is:

5 - oxo - L - prolyl - L - glutaminyl - L - aspartyl - L - (O - sulfato)tyrosyl - L - threonyl - glycyl - L - tryptophyl - L - methionyl - L - aspartyl - L - phenylalanine amide.

Attempts to synthesize caerulein have learnt that the main polypeptide chain of caerulein can easily be produced in accordance with conventional methods of peptide chemistry. The only remaining problem then is to introduce the O-sulfato group at the tyrosine residue in 4-position without simultaneously introducing a similar group at the threonine residue in 5-position of the polypeptide chain.

According to US—A—3 472 832 and Experientia 23, 700—702 (1967), the O-sulfato group can be introduced correctly by starting with $O^4$ - desulfato - $O^5$ - acetyl caerulein, treating this starting material with a pyridine/$SO_3$ complex in an anhydrous solvent and then deacetylating the resulting $O^5$-acetyl-caerulein. The resulting caerulein is useful as such or in the form of a pharmaceutically acceptable salt thereof.

Sulfating reactions effected on slightly different polypeptides but with the same pyridine/$SO_3$ complex in water or in anhydrous solvents are disclosed in GB—A—2 016 478 (example 4), US—A—3 892 726 (example 7) and FR—A—2 372 146.

However, the use of a pyridine/$SO_3$ complex for sulfating polypeptides has the disadvantage that side reactions may occur and that decomposition products of the complex will remain in solution, thus requiring complicated procedures (including countercurrent distribution steps) for isolating and purifying the desired product.

An object of the invention is to improve the prior art production process of caerulein and especially the sulfating reaction thereof in such a way that isolation and purification of the desired product can be accomplished easier and with higher yield and purity.

According to the invention, this object is achieved by using an insoluble complex of sulfur trioxide and a basic copolymer in an anhydrous solvent as an agent for effecting the sulfation reaction.

Contrary to a pyridine/$SO_3$ complex, the complex of $SO_3$ and a basic copolymer is insoluble in the anhydrous solvents as conventionally used. This means that the sulfation reaction of $O^4$ - desulfato - $O^5$ - acetylcaerulein can be effected under heterogeneous conditions and that after termination of the reaction, the remaining basic copolymer (from which the $SO_3$ has been consumed) as well as the unreacted complex may be removed quite easily by filtration. The filtrate can be concentrated under reduced pressure to give $O^5$-acetylcaerulein and this can be deacetylated in a known manner to give caerulein. The resulting caerulein may optionally be converted to a pharmaceutically acceptable salt thereof. According to the present invention, therefore, caerulein and its salts, can be produced without complicated isolation and purification steps as required in the prior art. The invented process is industrially applicable and provides an economical and simple manufacturing process.

It should be noted that the term $O^4$ - desulfato - $O^5$ - acetylcaerulein as used above, relates to a caerulein molecule wherein the sulfate ester group at the tyrosine residue in 4-position is absent and wherein the threonine residue in 5-position is protected by an acetyl group.

Further, it should be noted that a complex of sulfur trioxide and a basic homopolymer in water has been disclosed in US—A—3 057 855 as an agent for sulfating starch. This known complex was in water (column 3, line 3).

Reference is further made to EP—A—0 103 538 of slightly later date wherein insoluble sulfur trioxide complexes resembling those of the present invention are suggested for use in sulfonation and sulfating reactions of organic compounds.

In the following, the process of the present invention will be explained in detail.

As a basic copolymer, copolymers of vinylpyridines (e.g. 4-vinylpyridine, 2-methyl-5-vinylpyridine, etc. % taking advantage of its solubility or tert-amine-containing compounds such as aminoalkylated vinylbenzenes (e.g. 4-dimethylaminomethyl-vinylbenzene etc.) with divinylbenzene are employed. The ratio of the former to the latter is about 1:1 to 9:1, preferable 2:1 to 5:1, most preferably 3:1 to 4:1. These copolymers are available on the market as weak basic anion exchange resins. The resins having a capacity of about 3.0 to 6.0 meq/ml, especially 4.0 to 5.5 meq/ml are preferred. A microporous resin is favorable.

The complex of sulfur trioxide with a basic copolymer can be produced by adding sulfur trioxide to the above mentioned basic copolymer in an organic solvent under stirring. The production may be carried out in accordance with the method of Imoto et al. [Kogyo Kagaku Zasshi, Japan, vol. *65*, 1658 (1962)].

In the sulfation step of the present invention, $O^4$ - desulfato - $O^5$ - acetylcaerulein or its salts (inert to sulfation are employed as a starting material. The complex of sulfur trioxide and a basic copolymer is allowed to react with this starting material under ice-cooling or at room temperature, (preferably under ice-cooling), and in an anhydrous solvent. Solvents which are inert to the reaction such as e.g. dimethylformamide, dimethylsulfoxide, hexamethylphosphoric triamide, etc. can be selected; as reaction solvents and dimethylformamide is preferred in this respect. The reaction terminates within 1 to 20 hours.

2

**0 095 806**

After the termination of the reaction, the complex is removed by filtration and the solvent is distilled off from the filtrate under reduced pressure to give $O^5$-acetyl caerulein as a residue. Caerulein is obtained by deacetylation of this residue or alternatively by immediate treatment for deacetylation of the above filtrate without evaporation under reduced pressure. Deacetylation is conducted in a weakly basic aqueous solvent in a usual manner. When the desired end product is a diethylamine salt, it is profitable that the deacetylation is effected in an aqueous solution of diethylamine because contamination of impurities in the final product is decreased to a small degree then. The resulting caerulein is more than 90% pure and may be purified further, if desired, by chromatography or reprecipitation. Besides, when the deacetylation is effected with diethylamine and the resultant solution of diethylamine salt is adjusted to a pH of about 2.0 to 4.0 with, for example, acetic acid, diluted hydrochloric acid and the like, caerulein as a free base is precipitated in remarkably high purity. This purification procedure is very simple and effective.

If pharmaceutically acceptable salts are desired, the corresponding inorganic or organic bases are employed to convert the above obtained caerulein to such salts. In general, caerulein forms tribase addition salts and so, for example, an aqueous solution of diethylamine is added to an aqueous solution of caerulein to give caerulein tridiethylamine salt.

The following examples serve to illustrate the invention but are not intended to limit the scope thereof.

Example 1

(1) Preparation of a complex of sulfur trioxide with a basic copolymer

An anhydrous copolymer (10 g) of 4-vinylpyridine with divinylbenzene (manufactured by Kokei Kagaku Kogyo Co., Ltd. KEX-212, microporous type, capacity 4.8 meq/ml, 16—50 mesh) is swollen in a suspension of dry carbon tetrachloride (150 ml) under stirring at room temperature for 2 hours. Thereupon, sulfur trioxide (3.16 ml, 75.9 m mole) is added in small portions, and the mixture is stirred at room temperature for 20 hours. The resulting product is filtered off and dried under reduced pressure to give the desired complex (18.23 g) which contains 4.11 m mole/g of sulfur trioxide and 13.18% of sulfur, Elemental analysis: S, 12.85%).

(2) Preparation of caerulein

$O^4$-Desulfo-$O^5$-acetylcaerulein (0.984 g) is dissolved in dry dimethylformamide (59 ml). The complex (6 g) obtained in (1) is added thereto with stirring under ice-cooling, and the mixture is stirred for 10 hours at the same temperature. Then, the complex is collected by filtration and washed with dimethylformamide (50 ml). The filtrate and washings are combined, mixed with 1 M aqueous solution of diethylamine (75 ml), and then evaporated under reduced pressure. To the obtained residue a 1 M aqueous solution of diethylamine (75 ml) is added and allowed to stand at 3°C overnight.

The reaction mixture is evaporated under reduced pressure, the residue is dissolved in water (25 ml), and 1 N acetic acid (7 ml) is mixed therewith under ice-cooling. The precipitated solid is collected by filtration, dissolved in 0.1 M diethylamine (25 ml), chromatographed on an ion-exchange resin of the Trade Mark DEAE Sephadex (manufactured by Pharmacia AB), and eluted with 0.5—0.8 M aqueous solution of ammonium bicarbonate. The main eluate is evaporated under reduced pressure, and the residue is dissolved in water and lyophilized to give the title compound (0.891 g, yield: 88.0%).

The above product (0.80 g) is dissolved in water (11.8 ml) and the same volume of dimethylformamide is mixed therewith. Then, a swollen ion exchanger of the Trade Mark Dowex 50 W ($H^+$ type) (35.5 ml) is added thereto, and the resin is collected by filtration after stirring for 10 minutes. This resin is washed three times with water-dimethylformamide (1:1) (10 ml), and the filtrate and washings are combined, mixed with a 1 M aqueous solution (5.9 ml) of diethylamine, and evaporated under reduced pressure. The residue is dissolved in water, desalted on a column of Sephadex G 15, (Trade Mark) and then lyophilized to give the tridiethylamine salt of the title compound [0.834 g, $[\alpha]_D -22.8 \pm 0.6°$ ($H_2O$, c=1)]. This was identical with an authentic specimen in terms of infrared absorption spectrum and high-performance liquid chromatography.

Example 2

A copolymer of divinylbenzene and 4-dimethylaminomethyl - vinylbenzene (instead of 4-vinylpyridine) is used for preparing a complex with sulfur trioxide. The sulfation reaction is carried out at room temperature for 1 hour in the same manner as in Example 1 (2), and the reaction mixture is treated in the same manner to give caerulein tridiethylamine salt.

**Claims**

1. A process of producing caerulein or its salts, comprising the steps of sulfating $O^4$ - desulfato - $O^5$ - acetylcaerulein by reacting it with a complex of sulfur trioxide in an anhydrous solvent, deacetylating the resulting $O^5$-acetylcaerulein and optionally by converting the resulting caerulein to a pharmaceutically acceptable salt thereof, characterised in that the sulfation reaction is effected with an insoluble complex of sulfur trioxide and a basic copolymer in an anhydrous solvent.

2. The process as claimed in claim 1, wherein said basic copolymer is a copolymer of a vinylpyridine or an aminoalkylated vinylbenzene with divinylbenzene.

3

3. The process as claimed in claim 2, wherein said basic copolymer is a copolymer of 4-vinylpyridine or 4-dimethylaminomethyl-vinylbenzene with divinylbenzene.

4. The process as claimed in claim 2 or 3, wherein said basic copolymer is in the form of a weak basic anion exchange resin having a capacity of about 3.0 to 6.0 meq/ml.

5. A sulfating agent for caerulein which comprises complex of sulfur trioxide with a basic copolymer, that complex being insoluble in an anhydrous system.

6. The sulfating agent as claimed in claim 5, wherein said basic copolymer is a copolymer of a vinylpyridine or an aminoalkylated vinylbenzene with divinylbenzene.

7. The sulfating agent as claimed in claim 6, wherein said basic copolymer is a copolymer of 4-vinylpyridine or 4-dimethylaminomethyl-vinylbenzene with divinylbenzene.

8. The sulfating agent as claimed in claim 6 or 7, wherein said basic copolymer is in the form of a weak basic anion exchange resin having a capacity of 3.0 to 6.0 meq/ml.

**Patentansprüche**

1. Verfahren zur Herstellung von Caerulein oder seinen Salzen durch Sulfatisierung von $O^4$ - Desulfato - $O^5$ - acetylcaerulein durch Umsetzung eines Komplexes von Schwefeltrioxid in einem wasserfreien Lösungsmittel, Entacetylierung des entstehenden $O^5$-Acetylcaeruleins und gegebenenfalls Umwandlung des entstehenden Caeruleins in eines seiner pharmazeutisch annehmbaren Salze, dadurch gekennzeichnet, daß die Sulfatisierungsreaktion mit einem unlöslichen Komplex aus Schwefeltrioxid und einem basischen Copolymeren in einem wasserfreien Lösungsmittel durchgeführt wird.

2. Verfahren nach Anspruch 1, dadurch gekennzeichnet, daß das basische Copolymere ein Copolymeres aus Vinylpyridin oder einem aminoalkylierten Vinylbenzol mit Divinylbenzol ist.

3. Verfahren nach Anspruch 2, dadurch gekennzeichnet, daß das basische Copolymere ein Copolymeres aus 4-Vinylpyridin oder 4-Dimethylaminomethylvinylbenzol mit Divinylbenzol ist.

4. Verfahren nach Anspruch 2 oder 3, dadurch gekennzeichnet, daß das basische Copolymere in Form eines schwach basischen Anionenaustauscherharzes mit einer Kapazität von 3.0 bis 6.0 meq/ml vorliegt.

5. Sulfatisierungsmittel für Caerulein, dadurch gekennzeichnet, daß es einen Komplex aus Schwefeltrioxid mit einem basischen Copolymeren umfaßt, wobei der Komplex in einem wasserfreien System unlöslich ist.

6. Sulfatisierungsmittel nach Anspruch 5, dadurch gekennzeichnet, daß das basische Copolymere ein Copolymeres aus Vinylpyridin oder einem aminoalkylierten Vinylbenzol mit Divinylbenzol ist.

7. Sulfatisierungsmittel nach Anspruch 6, dadurch gekennzeichnet, daß das basische Copolymere ein Copolymeres aus 4-Vinylpyridin oder 4-Dimethylaminomethyl-vinylbenzol mit Divinylbenzol ist.

8. Sulfatisierungsmittel nach Anspruch 6 oder 7, dadurch gekennzeichnet, daß das basische Copolymere in Form eines schwach basischen Anionenaustauscherharzes mit einer Kapazität von 3.0 bis 6.0 meq/ml vorliegt.

**Revendications**

1. Procédé pour l'obtention de la caeruléine ou de ses sels, comprenant les étapes consistant à sulfater la $O^4$ - désulfato - $O^5$ - acétylcaeruléine en faisant réagir celle-ci avec un complexe de trioxyde de soufre dans un solvant anhydre, à désacétyler la $O^5$-acétylcaeruléine résultante et, de façon facultative, à convertir la caeruléine résultante en l'un de ses sels pharmaceutiquement acceptables, caractérisé par le fait que l'on effectue la réaction de sulfatation avec un complexe insoluble du trioxyde de soufre et d'un copolymère basique dans un solvant anhydre.

2. Procédé selon la revendication 1, dans lequel le copolymère basique est un copolymère d'une vinylpyridine ou d'un vinylbenzène aminoalkylé avec le divinylbenzène.

3. Procédé selon la revendication 2, dans lequel le copolymère basique est un copolymère de la vinyl-4 pyridine ou du diméthylaminométhyl-4 vinylbenzène avec le divinylbenzène.

4. Procédé selon la revendication 2 ou 3, dans lequel le copolymère basique se présente sous la forme d'une résine échangeuse d'anions base faible, présentant une capacité d'environ 3,0 à 6,0 méq/ml.

5. Agent de sulfatation pour la caeruléine qui consiste en un complexe de trioxyde de soufre avec un copolymère basique, ce complexe étant insoluble dans un système anhydre.

6. Agent de sulfatation selon la revendication 5, dans lequel le copolymère basique est un copolymère d'une vinylpyridine ou d'un vinylbenzène aminoalkylé avec le divinylbenzène.

7. Agent de sulfatation selon la revendication 6, dans lequel le copolymère basique est un copolymère de la vinyl-4 pyridine ou du diméthylaminométhyl-4 vinylbenzène avec le divinylbenzène.

8. Agent de sulfatation selon la revendication 6 ou 7, dans lequel le copolymère basique sa présente sous la forme d'une résine échangeuse d'anions base faible, présentant une capacité de 3,0 à 6,0 meq/ml.